# EUROPEAN PATENT APPLICATION

(11) **EP 3 933 043 A1**
(43) Date of publication of application: **05.01.2022**
(21) Application number: 20763387.6
(22) Date of filing: 26.02.2020
(51) Int. Cl.: C12P 17/00, C12P 41/00

(54) **METHOD FOR RESOLVING OPTICAL ISOMER BY MEANS OF ELECTRODIALYSIS TECHNIQUE**

(30) Priority: 27.02.2019 CN 201910146715
(71) Applicant: Guang an Mojia Biotechnology Co., Ltd., Guang An, Sichuan 638000 (CN)
(72) Inventor: CHIEW, Ansen, Shanghai 200120 (CN); SU, Jinhuan, Shanghai 200120 (CN); ZENG, Congming, Shanghai 200120 (CN); JIANG, Tailong, Shanghai 200120 (CN); CHEN, Yan, Shanghai 200120 (CN); LAU, Man Kit, Shanghai 200120 (CN)
(74) Representative: Straus, Alexander
(86) International application number: PCT/CN2020/076711
(87) International publication number: WO 2020/173453

(57) **Abstract**

Disclosed is a method for resolving an optical isomer from a racemate by means of electrodialysis. Specifically, an electrodialysis technique is used in an enzymatic resolution process, mainly in the separation of products after enzymatic resolution. Taking a preparation process for D-pantolactone as an example, the key point is that D-pantoic acid and L-pantolactone are separated from an enzymatic resolution solution by means of an electrodialysis method, which replaces the existing organic solvent extraction method. The process method is simple and easy to operate, has a high yield of D-pantoic acid of a good purity, greatly reduces the usage amount of an organic solvent, reduces production costs and is environmentally friendly, such that the working environment of workers can be improved to a great extent, and the operation safety index is improved.

## Description

### FIELD OF THE INVENTION

The present disclosure relates to the biotechnical field, and in particular, to resolution of optical isomers from racemates by using biocatalytic technique and electrodialysis technique.

### RELATED ART

Chirality is an essential attribute of nature, and many biological macromolecules and biologically active substances have chiral characteristics. Although the chemical compositions of two or more different configurations of a chiral substance are the same, the physiological activities are usually different. Usually only one configuration has the desired activity, and other configuration(s) has(ve) little or no effect, or may even have toxic side effects. For example, pantothenic acid is one of the B vitamins and a component of coenzyme A. It participates in metabolism of protein, fat, and sugar, and plays an important role in substance metabolism. Its active ingredient is dextrorotatory pantothenic acid (vitamin B5), which is D-configuration. However, because pantothenic acid is unstable, its commercial form is mainly calcium D-pantothenate.

Resolution is one of the main ways to obtain optically pure chiral compounds. Compared with conventional chemical resolution, enzymatic resolution does not require expensive resolution reagents, has mild reaction conditions, has good optical selectivity, is environmentally friendly, and can perform some reactions that cannot be performed by the chemical resolution. The enzymatic resolution has been increasingly popularized by scientific researchers from various countries due to its significant advantages, and there have been many successful industrialization cases.

For example, D-pantolactone is an important chiral intermediate to produce pantothenic acid series products such as calcium D-pantothenate, D-panthenol, and D-pantethine. At present, the industrial synthesis of D-pantolactone mostly uses a technical route that combining a chemical method with a hydrolase resolution method. That is, racemic DL-pantolactone is produced by the chemical method, and then is hydrolyzed and resolved with D-pantolactone hydrolase. L-pantolactone and unreacted D-pantolactone are first extracted from the supernatant after the resolution with an organic solvent, and the water phase (containing D-pantoic acid) is lactonized with acid and then extraction is performed with an organic solvent, and then the resulting product is desalted, decolorized, and refined by recrystallization. For example, CN1313402A discloses that DL-pantolactone is resolved by using free or immobilized cells, and then extraction is performed with dichloromethane, the water phase is acidified with hydrochloric acid, and then extraction is performed with dichloromethane, and the crude D-pantolactone obtained after solvent recovery is recrystallized in acetone/isopropyl ether to obtain qualified D-pantolactone. This process needs to be improved. For example, in the process of extracting and refining D-pantoic acid obtained by enzyme reaction, a large amount of organic solvents is used for extraction, which brings environmental and cost problems, and crude D-pantolactone needs to be recrystallized and refined, which has low yield and high cost.

Electrodialysis is an electrochemical separation process that separates electrolyte components from an aqueous solution by using ion-exchange membranes and a direct current electric field.

### SUMMARY OF THE INVENTION

The present disclosure provides a novel method for resolving an optical isomer. The method can remedy the defects of the existing chiral resolution process, and replaces a conventional organic solvent extraction process with an electrodialysis technique according to different ionizable degrees of optical isomers in a chiral resolution product, thereby improving the yield and product quality of a product, and reducing the production cost.

The present disclosure provides a method for resolving an optical isomer from a racemate by using electrodialysis, including:
a) reacting a racemate in the presence of the catalyst to form a mixture comprising an ionizable form of a first optical isomer and a non-ionized form of a second optical isomer;
b) electrodialyzing the mixture to allow the ionizable form of the first optical isomer and the non-ionized form of the second optical isomer to be separated; and
c) collecting the separated ionizable form of the first optical isomer, and/or collecting the separated non-ionized form of the second optical isomer.

In the present disclosure, the "racemate" refers to a mixture of two or more optical isomers with different optical rotation properties. For example, a compound with one chirality center may have two optical isomers, one with a chirality center in the R configuration, and the other with a chirality center in the S configuration. For the compound, its racemate includes both optical isomers in the R configuration and the S configuration. In the racemate of the present disclosure, different optical isomers may exist in equal molar mass (that is, optical rotation properties are offset), or may exist in unequal molar mass.

In some embodiments, the racemate has a hydrolyzable functional group. The hydrolyzable functional group includes, for example, but not limited to, an ester bond or an amide bond, etc. In some embodiments, the functional group may be hydrolyzed to form an ionizable group. The ionizable group refers to a group that can be ionized in an aqueous solution, such as a carboxyl group, an amino group, etc. The ionizable group produces charged groups after ionization, such as a negatively charged carboxylate, a positively charged ammonia ion, etc. In some embodiments, the chirality center in the racemate may be located within the hydrolyzable functional group, or may be located near the hydrolyzable functional group, for example, on the atom adjacent to the hydrolyzable functional group, or at a position separated from the hydrolyzable functional group by 1, 2, or 3 atoms.

In the method of the present disclosure, the catalyst may specifically react with a specific optical isomer in the racemate (for example, hydrolyze the hydrolyzable functional group therein) to covert the optical isomer into an ionizable form. The "ionizable form" in the present disclosure refers to that an optical isomer can be ionized in an aqueous solution to form charged groups. In some embodiments, the ionizable form may include ionizable groups, such as a carboxyl group, an amino group, etc. In some embodiments, the catalyst may not catalyze the second optical isomer in the racemate to keep it in a non-ionized form. The "non-ionized form" in the present disclosure refers to that an optical isomer can neither be ionized in an aqueous solution, nor have charged groups. In some embodiments, the non-ionized form includes non-ionized groups, such as ester (for example, lactone in a racemate), amide, ether, etc.

In some embodiments, the racemate has a ring structure, and the hydrolyzable functional group is within the ring structure. For example, an exemplary ring structure is lactone or lactam. These ring functional groups may react to open the ring. In some embodiments, the ring structure in the non-ionized form of the second optical isomer is closed. In some embodiments, the ring structure is ring-opened in the ionizable form of the first optical isomer. For example, the ring functional group undergoes a ring-opening reaction to form ionizable groups. Alternatively, In some embodiments, the ring structure is ring-opened in the non-ionized form of the second optical isomer, and/or the ring structure in the ionizable form of the first optical isomer is closed. The chirality center of a racemate with a ring structure may or may not be on a ring atom.

In some embodiments, the racemate is ester. An exemplary racemic ester includes methyl 3-cyclohexene-1-carboxylate. In some embodiments, the racemate is lactone. The lactone has an intramolecular ester bond (-C(O)O) formed by dehydration of carboxyl and hydroxyl groups in the molecular structure. The intramolecular ester bond is usually in the ring structure. Examples of lactone include, for example, racemic DL-pantolactone, β-butyrolactone, γ-butyrolactone, α-hydroxy-γ-butyrolactone, β-hydroxy-γ-butyrolactone, α-acetyl-γ-butyrolactone, n-butylphthalide, etc.

In some embodiments, the catalyst comprises an enzyme composition. In some embodiments, the enzyme composition comprises an enzyme that can specifically react with a certain optical isomer. For example, the enzyme specifically reacts with a D-configuration optical isomer, or specifically reacts with an L-configuration optical isomer. In some embodiments, the enzyme composition comprises an ester hydrolase. In some embodiments, the ester hydrolase specifically catalyzes D-configuration lactone. Examples of ester hydrolase include D-pantolactone hydrolase, Novozyme 435 lipase, β-butyrolactone hydrolase, γ-butyrolactone hydrolase, α-hydroxy-γ-butyrolactone hydrolase, β-hydroxy-γ-butyrolactone hydrolase, α-acetyl-γ-butyrolactone hydrolase, n-butylphthalide hydrolase, etc. For example, the D-pantolactone hydrolase can specifically hydrolyze D-configuration pantolactone in the racemate, so that the lactone structure thereof is hydrolyzed to form intramolecular independent carboxyl and hydroxyl groups. The carboxyl group can be ionized in an aqueous solution, so that the D-configuration pantolactone can be charged and is in an ionizable form. However, the D-pantolactone hydrolase cannot hydrolyze L-configuration pantolactone in the racemate, so that the L-configuration pantolactone still remains the lactone structure after a catalytic reaction, which is in a non-ionized form. In another example, the Novozyme 435 lipase can specifically hydrolyze R-configuration methyl 3-cyclohexene-1-carboxylate in the racemate to form 3-cyclohexene-1-carboxylic acid, which can be ionized in an aqueous solution. S-configuration methyl 3-cyclohexene-1-carboxylate cannot be hydrolyzed, so that it still remains in a non-ionized form.

In some embodiments, the enzyme composition comprises lactamase. In some embodiments, the lactamase specifically catalyzes D-configuration lactam. Examples of lactamase includes, for example, β-lactamase or γ-lactamase. For example, the β-lactamase can specifically hydrolyze D-configuration β-lactam in the racemate, so that the lactam structure thereof is hydrolyzed to form intramolecular independent carboxyl and amino groups. The carboxyl group can be ionized in an aqueous solution, so that the D-configuration pantolactone can be charged and is in an ionizable form. However, the β-lactamase cannot hydrolyze L-configuration β-lactam in the racemate, so that the L-configuration β-lactam still remains the lactam structure after a catalytic reaction, which is in a non-ionized form.

In some embodiments, in the present disclosure, the racemate is DL-pantolactone, the first optical isomer is D-pantolactone, the second optical isomer is L-pantolactone, the ionizable form of the first optical isomer is D-pantoic acid, and the non-ionized form of the second optical isomer is L-pantolactone.

In some embodiments, the racemate is methyl 3-cyclohexene-1-carboxylate, the first optical isomer is (R)-methyl 3-cyclohexene-1-carboxylate, the second optical isomer is (S)-methyl 3-cyclohexene-1-carboxylate, the ionizable form of the first optical isomer is (R)-3-cyclohexene-1-carboxylic acid, and the non-ionized form of the second optical isomer is (S)-methyl 3-cyclohexene-1-carboxylate.

In some embodiments, the racemate is α-hydroxy-γ-butyrolactone, the first optical isomer is (R)-α-hydroxy-γ-butyrolactone, the second optical isomer is (S)-α-hydroxy-γ-butyrolactone, the ionizable form of the first optical isomer is (R)-α-hydroxy-γ-butyric acid, and the non-ionized form of the second optical isomer is (S)-α-hydroxy-γ-butyrolactone.

In some embodiments, the racemate is β-hydroxy-γ-butyrolactone, the first optical isomer is (R)-β-hydroxy-γ-butyrolactone, the second optical isomer is (S)-β-hydroxy-γ-butyrolactone, the ionizable form of the first optical isomer is (R)-β-hydroxy-γ-butyric acid, and the non-ionized form of the second optical isomer is (S)-β-hydroxy-γ-butyrolactone.

In some embodiments, the racemate is α-acetyl-γ-butyrolactone, the first optical isomer is (R)-α-acetyl-γ-butyrolactone, the second optical isomer is (S)-α-acetyl-γ-butyrolactone, the ionizable form of the first optical isomer is (R)-α-acetyl-γ-butyric acid, and the non-ionized form of the second optical isomer is (S)-α-acetyl-γ-butyrolactone.

Any form of enzyme with a selective catalytic function for optical isomers may be used. In some embodiments, the enzyme composition may comprise purified enzyme, enzyme-expressing cells, or enzyme-expressing lysates. The cells expressing the enzyme may be any suitable host cells, which may be prokaryotic cells, for example, bacteria, or may be eukaryotic cells, such as yeast and animal cells. The lysates of cells may be any lysate components containing enzyme, for example, cell lysis solution. In some embodiments, the enzyme composition is immobilized on a substrate. A suitable substrate may include materials for immobilizing enzyme, such as magnetic microspheres and macroporous resins, or may include materials for immobilizing cells, such as calcium alginate and gels.

In some embodiments, in step a), the pH value is maintained within the range of 7.0-7.5 during reaction, for example, the pH value is maintained at 7.0, 7.1, 7.2, 7.3, 7.4, 7.5, or any value between any two of the foregoing values. In some embodiments, 15N NH₃·H₂O is used for titration to maintain the pH value. In some embodiments, in step a), the temperature is maintained between 20°C and 40°C during reaction, for example, 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, 40°C, or any value between any two of the foregoing values. In some embodiments, in step a), the reaction time is 1-10 h, for example, 1 h, 2 h, 3 h, 4 h, 5 h, 6 h, 7 h, 8 h, 9 h, 10 h, or any value between any two of the foregoing values.

In some embodiments, after step a) and before step b), the method further comprises removing a residue of the catalyst in the mixture. The residue includes macromolecules such as cell debris and proteins. A person skilled in the art may remove the residue of the catalyst in the mixture according to actual needs by using conventional separation methods, for example, one or more of a plurality of methods such as filtration, centrifugation, microfiltration, and ultrafiltration.

In some embodiments, the filtration is performed by using filter paper or filter cloth. The filter paper or filter cloth in the present disclosure could be commercially available filter paper or filter cloth, for example, filter paper or filter cloth produced by companies such as GE Healthcare Life Sciences, Spectrum Laboratories Inc., and Asahi KASEI. In some embodiments, the pore size of the filter paper or filter cloth is 10-150 µm, for example, 10 µm, 20 µm, 30 µm, 40 µm, 50 µm, 60 µm, 70 µm, 80 µm, 90 µm, 100 µm, 110 µm, 120 µm, 130 µm, 140 µm, 150 µm, or any value between any two of the foregoing values. A person skilled in the art may select a suitable pore size of the filter paper or filter cloth to remove the residue of the catalyst according to the type and size of the residue of the catalyst.

In some embodiments, the centrifugation is performed by using a centrifugal separator. The centrifugal separator in the present disclosure could be a commercially available centrifugal separator, for example, a centrifugal separator produced by companies such as Guangzhou Fuyi Liquid Separation Technology Co., Ltd., Yantai Chengbo Machinery Technology Co., Ltd., Dongguan Yaotian Electric Technology Co., Ltd., TEMA System, Kyte, Heinkel, and GEA. In some embodiments, the centrifugal rate is 1000-2000 rpm, for example, 1000 rpm, 1100 rpm, 1200 rpm, 1300 rpm, 1400 rpm, 1500 rpm, 1600 rpm, 1700 rpm, 1800 rpm, 1900 rpm, 2000 rpm, or any value between any two of the foregoing values. In some embodiments, the centrifugal time is 2-15 min, for example, 2 min, 3 min, 4 min, 5 min, 6 min, 7 min, 8 min, 9 min, 10 min, 11 min, 12 min, 13 min, 14 min, 15 min, or any value between any two of the foregoing values. A person skilled in the art may select a suitable centrifugal rate and centrifugal time to remove the residue of the catalyst according to the type and size of the residue of the catalyst.

In some embodiments, the microfiltration is performed by passing the mixture through a microfiltration membrane. The microfiltration membrane in the present disclosure could be a commercially available microfiltration membrane, for example, a hollow fiber microfiltration membrane series produced by companies such as GE Healthcare Life Sciences, Spectrum Laboratories Inc., and Asahi KASEI. In some embodiments, the pore size of the microfiltration membrane is 0.1-0.6 µm, for example, 0.1 µm, 0.15 µm, 0.2 µm, 0.22 µm, 0.25 µm, 0.3 µm, 0.35 µm, 0.4 µm, 0.45 µm, 0.5 µm, 0.55 µm, 0.6 µm, or any value between any two of the foregoing values. According to the size of the residue of the catalyst, selecting the pore size as small as possible of the microfiltration membrane helps to remove large-particle residues.

In some embodiments, the ultrafiltration is performed by passing the mixture through an ultrafiltration membrane. The ultrafiltration membrane in the present disclosure could be a commercially available ultrafiltration membrane, for example, a hollow fiber ultrafiltration membrane series produced by companies such as GE Healthcare Life Sciences, Spectrum Laboratories Inc., and Asahi KASEI. In some embodiments, the ultrafiltration membrane is a hollow fiber ultrafiltration membrane with a pore size of 10-500 kD, for example, a hollow fiber ultrafiltration membrane with a pore size of 10 kD, 20 kD, 30 kD, 40 kD, 50 kD, 60 kD, 70 kD, 80 kD, 90 kD, 100 kD, 150 kD, 200 kD, 250 kD, 300 kD, 350 kD, 400 kD, 450 kD, 500 kD, or any value between any two of the foregoing values. A person skilled in the art may select a suitable pore size of the ultrafiltration membrane to remove the residue of the catalyst according to the size of the residue of the catalyst.

In some embodiments, the method of the present disclosure further comprises purifying and/or concentrating the separated ionizable form of the first optical isomer, and/or purifying and/or concentrating the separated non-ionized form of the second optical isomer.

In some embodiments, the separated ionizable form of the first optical isomer and/or non-ionized form of the second optical isomer may be further purified. For example, the ionizable form of the first optical isomer and/or the non-ionized form of the second optical isomer may be extracted by using a suitable solvent. For example, an organic solvent (for example, ethyl acetate) may be added into the collected (R)-3-cyclohexene-1-carboxylic acid, and the organic phase may be collected, to obtain the purified (R)-3-cyclohexene-1-carboxylic acid. In another example, an organic solvent (for example, ethyl acetate) may be further added into the collected (S)-methyl 3-cyclohexene-1-carboxylate, and the organic phase may be collected, to obtain the purified (S)-methyl 3-cyclohexene-1-carboxylate.

In some embodiments, the separated and/or purified ionizable form of the first optical isomer and/or non-ionized form of the second optical isomer may be further concentrated. In some embodiments, the concentration is performed by reducing pressure. For example, the separated and/or purified ionizable form of the first optical isomer and/or the separated and/or purified non-ionized form of the second optical isomer are/is pumped into a concentration equipment for concentration under reduced pressure.

In some embodiments, the present disclosure further comprises converting the non-ionized form of the second optical isomer into the racemate. The non-ionized form of the second optical isomer could be the substance separated by the method provided in the present disclosure, or may be further purified, or may be further concentrated. For example, when the racemate is an ester, the separated non-ionized form (that is, the ester) of the second optical isomer may be racemized to obtain the racemate with different chiral isomers. By reconverting the resolved second optical isomer into the racemate, the chiral resolution may be further performed by the method provided in the present disclosure to obtain more first optical isomers.

In some embodiments, the present disclosure further includes converting the separated ionizable form of the first optical isomer into the non-ionized form. In some embodiments, the separated (and/or purified or concentrated) ionizable form of the first optical isomer may be further reacted to restore the ionizable groups therein to hydrolyzable functional groups. For example, in some embodiments, the separated ionizable form of the first optical isomer is D-pantoic acid, which may be lactonized to obtain D-pantolactone, so that the ionizable group (i.e. carboxyl group) therein is restored to the hydrolyzable functional group (i.e. lactone).

A person skilled in the art may use known methods and equipment to perform the electrodialysis step in the method of the present disclosure. For the electrodialysis equipment and methods, refer to Industry Standard of the People's Republic of China-Electrodialysis Technology HY/T 034.1-034.5-1994.

In some embodiments, the electrodialyzing is carried out in an electrodialysis cell, and the electrodialysis cell has a dilute compartment and a concentrate compartment separated by ion-exchange membranes. In some embodiments, the ion-exchange membrane is a homogeneous membrane or a heterogeneous membrane. In the electrodialyzing process, driven by an external electric field, anions and cations move to an anode and a cathode respectively according to permselectivity of the ion-exchange membrane (for example, cations can pass through the cation-exchange membrane, and anions can pass through the anion-exchange membrane).

A person skilled in the art may select various ion-exchange membranes known in the art for electrodialysis according to actual needs. In some embodiments, the ion-exchange membrane is an anion-exchange membrane, for example, a Q membrane. In some embodiments, the ion-exchange membrane is a cation-exchange membrane, for example, an S membrane. In some embodiments, the ion-exchange membranes are a cation-exchange membrane and an anion-exchange membrane. In some embodiments, the cation-exchange membrane allows the transport of cations, while repels and blocks the transport of anions. In some embodiments, the anion-exchange membrane allows the transport of anions, while repels and blocks the transport of cations. In some embodiments, a compartment formed between the cation-exchange membrane and the anode, and between the anion-exchange membrane and the cathode is a concentrate compartment, and a compartment formed between the cation-exchange membrane and the anion-exchange membrane is a dilute compartment. In some embodiments, the cation-exchange membrane and the anion-exchange membrane are commercially available, for example, from Novasep, Eurodia, Shandong Tianwei Membrane Technology Co., Ltd., or Zhejiang Qianqiu Environmental Protection Water Treatment Co., Ltd.

In some embodiments, a person skilled in the art may select the size of a membrane stack of the homogeneous membrane or the heterogeneous membrane according to actual needs, for example, 10*20 cm, 10*30 cm, or 20*30 cm. In some embodiments, a person skilled in the art may select the number of membrane pairs of the homogeneous membrane or the heterogeneous membrane according to actual needs, for example, 5 pairs, 10 pairs, 15 pairs, or 20 pairs.

In some embodiments, the electrodialyzing comprises placing the mixture in the dilute compartment, placing a solvent in the concentrate compartment, and energizing the electrodialysis cell to allow the ionizable form of the first optical isomer in the dilute compartment to migrate into the solvent in the concentrate compartment.

In some embodiments, in the electrodialyzing process, the flow rate is adjusted to adjust the pressure of the concentrate compartment and the dilute compartment, so that the pressure of the concentrate compartment divided by the pressure of the dilute compartment is 1, 2, 3, 4, 5, or any value between any two of the foregoing values. In some embodiments, the electrodialyzing is carried out under a constant voltage until the electrical conductivity of the dilute compartment is less than 30 µs/cm, 40 µs/cm, 50 µs/cm, 60 µs/cm, 70 µs/cm, 80 µs/cm, 90 µs/cm, 100 µs/cm, 110 µs/cm, 120 µs/cm, 130 µs/cm, 140 µs/cm, or 150 µs/cm. In some embodiments, the constant voltage is 10V, 15V, 20V, 25V, 30V, 35V, 40V, 45V, or 50V.

In some embodiments, the solvent includes pure water.

In some embodiments, the electrodialyzing is carried out in one electrodialysis cell. In some embodiments, step b) of the method of the present disclosure may be repeated in the electrodialysis cell, thereby improving the separation efficiency. For example, a supernatant of the concentrate compartment may be pumped into the dilute compartment of the electrodialysis cell in an electrodialysis device to repeat the electrodialysis step in the electrodialysis cell.

In some embodiments, the electrodialyzing is carried out in more than one electrodialysis cells that are connected in series. For example, a supernatant of the concentrate compartment may be pumped into dilute compartments of second stage, third stage, fourth stage, and even more stage of electrodialysis devices to repeat step b) of the method of the present disclosure, thereby improving the separation efficiency. In some embodiments, the pressures of the concentrate compartment and the dilute compartment between different electrodialysis cells are the same. In some embodiments, the pressures of the concentrate compartment and the dilute compartment between different electrodialysis cells are different. In some embodiments, the voltages between different electrodialysis cells are the same. In some embodiments, the voltages between different electrodialysis cells are different.

The purity of the optical isomer obtained through resolution by the method of the present disclosure is greater than 90%, for example, greater than 91%, 92%, 93%, 94%, 95%, 96%, 97%, 98%, or 99%, or even 100%. In some embodiments, the purity of the optical isomer obtained through resolution by the method of the present disclosure is represented by an ee value. A person skilled in the art may measure or calculate the ee value according to conventional technical means (for example, the HPLC method) in the art. For example, if a racemate contains two optical isomers A and B, ee value =A% - B%.

Compared with the related art, the present disclosure at least has the following advantages:
1. One of the advantages of the present disclosure is combining the biocatalysis (for example, enzyme catalysis) technique with the electrodialysis technique, and resolving the optical isomer in the racemate by using the electrodialysis technique according to different ionization degrees of the product produced by the enzyme catalysis, which has mild reaction conditions and reduces the operation steps.
2. The electrodialysis technique replaces conventional extraction methods, for example, conventional organic solvent extraction, which greatly reduces the amount of an organic solvent, reduces production costs, and reduces environmental pollution.
3. The present disclosure improves the extraction rate of the product, has good product purity so the product can be directly applied without further refining, reduces procedures, and has more cost advantages.
4. The process is simple and easy to implement, which facilitates automatic operation, improves the operation safety index, and improves the working environment of workers.

### DETAILED DESCRIPTION

The present disclosure is further described below with reference to specific examples, but the protection scope of the present disclosure is not limited thereto.

### Example 1

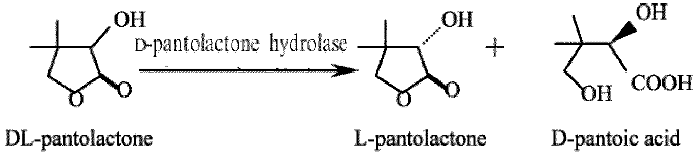

1. Preparation of an enzymatic conversion solution: 600 g of racemic pantolactone and 300 g of immobilized cells containing D-pantolactone hydrolase were added into a 2 L system at 30°C with a pH of 7.0, the mixture was mechanically stirred at 200 rpm, and was titrated with 15N NH₃·H₂O to keep the pH value at 7.0, to react for 3 h.
2. Pretreatment of the enzymatic conversion solution: the enzymatic conversion solution was first filtered with a filter cloth, then filtered with a 0.2 µm microfiltration membrane, and then filtered with a 50 kD ultrafiltration membrane.
3. Electrodialysis separation: A homogeneous membrane stack B (size: 10*30 cm; number of membrane pairs: 5 pairs) was used, a supernatant of the ultrafiltrate was pumped into an electrodialysis dilute compartment, 2 L of pure water was added into a concentrate compartment, the flow rate was adjusted to equalize the pressures of three compartments, and the electrodialysis was performed at a constant voltage of 10 V until the electrical conductivity of the dilute compartment was < 100 µs/cm.
   A supernatant of the concentrate compartment was pumped into a dilute compartment of a second-stage electrodialysis device, 2 L of pure water was added into a concentrate compartment, the flow rate was adjusted to equalize the pressures of three compartments, and the electrodialysis was performed at a constant voltage of 10 V until the electrical conductivity of the dilute compartment was < 100 µs/cm.
4. Concentration and acidification: A supernatant of the electrodialysis concentrate compartment was injected into a concentration equipment to be concentrated to about 400 mL under reduced pressure, and sulfuric acid was added into the concentrated supernatant to about pH1 to lactonize it.
5. Crystallization: After the concentration, an upper layer of the acidified solution was removed to obtain 259.2 g of D-pantolactone with a yield of 43.2% (based on DL-pantolactone), and the ee value of the D-pantolactone measured by HPLC was 98.9%.

### Example 2

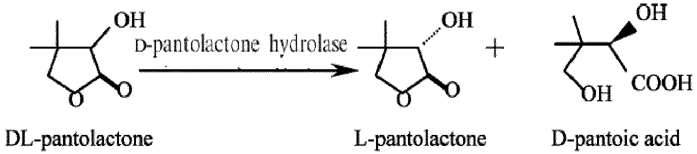

1. Preparation of an enzymatic conversion solution: 900 g of racemic pantolactone and 90 g of cells containing D-pantolactone hydrolase were added into a 3 L system at 30°C with a pH of 7.0, the mixture was mechanically stirred at 200 rpm, and was titrated with 15N NH₃·H₂O to keep the pH value at 7.0, to react for 5 h.
2. Pretreatment of the enzymatic conversion solution: the enzymatic conversion solution was first centrifuged with a butterfly centrifuge, then filtered with a 0.4 µm microfiltration membrane, and then filtered with a 20 kD ultrafiltration membrane.
3. Electrodialysis separation: A heterogeneous membrane stack Z (size: 10*20 cm; number of membrane pairs: 10 pairs) was used, a supernatant of the ultrafiltrate was pumped into an electrodialysis dilute compartment, 3 L of pure water was added into a concentrate compartment, the flow rate was adjusted to make the pressure of the concentrate compartment 3 times that of the dilute compartment, and the electrodialysis was performed at a constant voltage of 25 V until the electrical conductivity of the dilute compartment was < 100 µs/cm.
   A supernatant of the concentrate compartment was pumped into a dilute compartment of a second-stage electrodialysis device, 3 L of pure water was added into a concentrate compartment, the flow rate was adjusted to make the pressure of the concentrate compartment 3 times that of the dilute compartment, and the electrodialysis was performed at a constant voltage of 25 V until the electrical conductivity of the dilute compartment was < 100 µs/cm.
   A supernatant of the concentrate compartment was pumped into a dilute compartment of a third-stage electrodialysis device, 3 L of pure water was added into a concentrate compartment, the flow rate was adjusted to make the pressure of the concentrate compartment 3 times that of the dilute compartment, and the electrodialysis was performed at a constant voltage of 25 V until the electrical conductivity of the dilute compartment was < 100 µs/cm.
   A supernatant of the concentrate compartment was pumped into a dilute compartment of a fourth-stage electrodialysis device, 3 L of pure water was added into a concentrate compartment, the flow rate was adjusted to make the pressure of the concentrate compartment 3 times that of the dilute compartment, and the electrodialysis was performed at a constant voltage of 25 V until the electrical conductivity of the dilute compartment was < 100 µs/cm.
4. Concentration and acidification: a supernatant of the electrodialysis concentrate compartment was injected into a concentration equipment to be concentrated to about 500 mL under reduced pressure, and sulfuric acid was added into the concentrated supernatant to about pH1 to lactonize it.
5. Crystallization: After the concentration, an upper layer of the acidified solution was removed to obtain 364.5 g of D-pantolactone with a yield of 40.5% (based on DL-pantolactone), and the ee value of the D-pantolactone measured by HPLC was 97.6%.

### Example 3

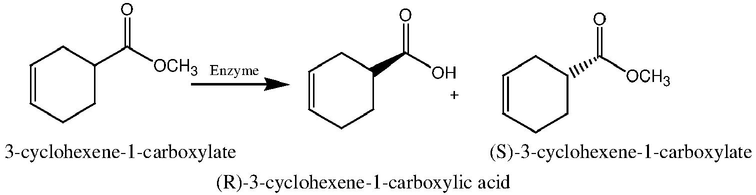

1. Preparation of an enzymatic conversion solution: 20 mL of methyl 3-cyclohexene-1-carboxylate and 10 g of Novozyme 435 lipase were added into a 1 L system at 35°C with a pH of 7.5, the mixture was mechanically stirred at 200 rpm, and was titrated with 1N NaOH to keep the pH value at 7.5, to react for 5 h.
2. Pretreatment of the enzymatic conversion solution: the enzymatic conversion solution was first filtered with a filter paper, and then filtered with a 0.4 µm microfiltration membrane.
3. Electrodialysis separation: a homogeneous membrane stack S (size: 10*20 cm; number of membrane pairs: 10 pairs) was used, a supernatant of the ultrafiltrate was pumped into an electrodialysis dilute compartment, 1 L of pure water was added into a concentrate compartment, the flow rate was adjusted to equalize the pressures of three compartments, and the electrodialysis was performed at a constant voltage of 14 V until the electrical conductivity of the dilute compartment dropped to < 100 µs/cm.
   A supernatant of the concentrate compartment was pumped into a dilute compartment of a second-stage electrodialysis device, 1 L of pure water was added into a concentrate compartment, the flow rate was adjusted to equalize the pressures of three compartments, and the electrodialysis was performed at a constant voltage of 14 V until the electrical conductivity of the dilute compartment was < 100 µs/cm.
   A supernatant of the concentrate compartment was pumped into a dilute compartment of a third-stage electrodialysis device, 1 L of pure water was added into a concentrate compartment, the flow rate was adjusted to equalize the pressures of three compartments, and the electrodialysis was performed at a constant voltage of 14 V until the electrical conductivity of the dilute compartment was < 100 µs/cm.
4. Treatment of a supernatant of the concentrate compartment: a supernatant of the electrodialysis concentrate compartment was injected into a concentration equipment to be concentrated to about 350 mL under reduced pressure, sulfuric acid was added into the concentrated supernatant to about pH5, an equal volume of ethyl acetate was added to extract and collect an organic phase, and distillation was performed under reduced pressure, to obtain (R)-3-cyclohexene-1-carboxylic acid with an ee value > 99% and a yield of about 25.2%.
5. Treatment of a supernatant of the dilute compartment: supernatants of the three stages of dilute compartments were mixed and injected into a concentration equipment to be concentrated to about 400 mL under reduced pressure, an equal volume of ethyl acetate was added to extract and collect an organic phase, and distillation was performed under reduced pressure, to obtain (S)-methyl 3-cyclohexene-1-carboxylate with an ee of 74.1% and a yield of about 41.2%.

## Claims

1. A method for resolving an optical isomer from a racemate by using electrodialysis, comprising:
a) reacting the racemate in the presence of a catalyst to form a mixture comprising an ionizable form of a first optical isomer and a non-ionized form of a second optical isomer;
b) electrodialyzing the mixture to allow the ionizable form of the first optical isomer and the non-ionized form of the second optical isomer to be separated; and
c) collecting the separated ionizable form of the first optical isomer, and/or collecting the separated non-ionized form of the second optical isomer.

2. The method according to claim 1, wherein the racemate has a hydrolyzable functional group.

3. The method according to claim 2, wherein the functional group is hydrolyzed to form an ionizable group.

4. The method according to claim 2 or 3, wherein the catalyst specifically hydrolyzes the hydrolyzable functional group of the first optical isomer to form the ionizable form of the first optical isomer.

5. The method according to any one of claims 1 to 4, wherein the racemate has a ring structure, and the hydrolyzable functional group is within the ring structure.

6. The method according to claim 5, wherein the ring structure is selected from the group consisting of a lactone and a lactam.

7. The method according to claim 5 or 6, wherein the ring structure is ring-opened in the ionizable form of the first optical isomer.

8. The method according to any one of claims 1 to 7, wherein the catalyst comprises an enzyme composition.

9. The method according to claim 8, wherein the enzyme composition comprises an ester hydrolase and/or a lactamase.

10. The method according to claim 8 or 9, wherein the enzyme composition comprises a purified enzyme, an enzyme-expressing cell, or an enzyme-expressing cell lysate.

11. The method according to any one of claims 8 to 10, wherein the enzyme composition is immobilized on a substrate.

12. The method according to any one of claims 1 to 11, wherein after step a) and before step b), the method further comprising removing a residue of the catalyst in the mixture.

13. The method according to any one of claims 1 to 12, further comprising purifying and/or concentrating the separated ionizable form of the first optical isomer, and/or purifying and/or concentrating the separated non-ionized form of the second optical isomer.

14. The method according to any one of claims 1 to 13, further comprising converting the non-ionized form of the second optical isomer into the racemate.

15. The method according to any one of claims 1 to 14, wherein the electrodialyzing is carried out in an electrodialysis cell, wherein the electrodialysis cell comprises a dilute compartment and a concentrate compartment separated by an ion-exchange membrane.

16. The method according to claim 15, wherein the electrodialyzing comprises placing the mixture in the dilute compartment, placing a solvent in the concentrate compartment, and energizing the electrodialysis cell to allow the ionizable form of the first optical isomer in the dilute compartment to migrate into the solvent in the concentrate compartment.

17. The method according to claim 16, wherein the solvent comprises pure water.

18. The method according to any one of claims 15 to 17, wherein the ion-exchange membrane is a homogeneous membrane or a heterogeneous membrane.

19. The method according to any one of claims 15 to 18, wherein the electrodialyzing is carried out in one electrodialysis cell or in more than one electrodialysis cells that are connected in series.

20. The method according to any one of claims 1 to 19, wherein the racemate is DL-pantolactone, the ionizable form of the first optical isomer is D-pantoic acid, and the non-ionized form of the second optical isomer is L-pantolactone.

21. The method according to any one of claims 1 to 19, wherein the racemate is methyl 3-cyclohexene-1-carboxylate, the ionizable form of the first optical isomer is (R)-3-cyclohexene-1-carboxylic acid, and the non-ionized form of the second optical isomer is (S)-methyl 3-cyclohexene-1-carboxylate.

22. The method according to any one of claims 1 to 19, wherein the racemate is α-hydroxy-γ-butyrolactone, the ionizable form of the first optical isomer is (R)-α-hydroxy-γ-butyric acid, and the non-ionized form of the second optical isomer is (S)-α-hydroxy-γ-butyrolactone.

23. The method according to any one of claims 1 to 19, wherein the racemate is β-hydroxy-γ-butyrolactone, the ionizable form of the first optical isomer is (R)-β-hydroxy-γ-butyric acid, and the non-ionized form of the second optical isomer is (S)-β-hydroxy-γ-butyrolactone.

24. The method according to any one of claims 1 to 19, wherein the racemate is α-acetyl-γ-butyrolactone, the ionizable form of the first optical isomer is (R)-α-acetyl-γ-butyric acid, and the non-ionized form of the second optical isomer is (S)-α-acetyl-γ-butyrolactone.

25. The method according to any one of claims 1 to 24, wherein the catalyst is D-pantolactone hydrolase, Novozyme 435 lipase, α-hydroxy-γ-butyrolactone hydrolase, β-hydroxy-γ-butyrolactone hydrolase, or α-acetyl-γ-butyrolactone hydrolase.
